# EUROPEAN PATENT SPECIFICATION

(11) **EP 3 813 927 B1**
(45) Date of publication and mention of the grant of the patent: **12.06.2024**
(21) Application number: 19769295.7
(22) Date of filing: 29.08.2019
(51) Int. Cl.: A61N 1/32, A61N 1/05, A61B 18/14

(54) **COMBINATION DENERVATION THERAPY FOR GLUCOSE CONTROL IN METABOLIC DISORDERS**
KOMBINATIONSDENERVIERUNGSTHERAPIE ZUR GLUCOKEKONTROLLE BEI STOFFWECHSELERKRANKUNGEN
POLYTHÉRAPIE DE DÉNERVATION POUR LA RÉGULATION DU GLUCOSE DANS DES TROUBLES MÉTABOLIQUES

(30) Priority: 29.08.2018 US 201862724233 P; 28.08.2019 US 201916553640
(43) Date of publication of application: 05.05.2021
(73) Proprietor: Boston Scientific Scimed Inc., Maple Grove, Minnesota 55311 (US)
(72) Inventor: CLARK, Bryan Allen, Forest Lake, Minnesota 55025 (US); KOYA, Vijay, Blaine, Minnesota 55449 (US); ANNONI, Elizabeth M., White Bear Lake, Minnesota 55110 (US); CAO, Hong, Maple Grove, Minnesota 55311 (US)
(74) Representative: Vossius & Partner Patentanwälte Rechtsanwälte mbB
(86) International application number: PCT/US2019/048813
(87) International publication number: WO 2020/047256

(56) References cited:
- WO-A1-2010/057043
- US-A1- 2012 259 269
- US-A1- 2014 018 880
- HANSLICK J L ET AL: "Dimethyl sulfoxide (DMSO) produces widespread apoptosis in the developing central nervous system", NEUROBIOLOGY OF DISEASE, ELSEVIER, AMSTERDAM, NL, vol. 34, no. 1, 1 April 2009 (2009-04-01), pages 1-10, XP026031294, ISSN: 0969-9961, DOI: 10.1016/J.NBD.2008.11.006 [retrieved on 2008-12-03]

## Description

### FIELD

The present disclosure relates to methods, devices, kits and systems for enhancing the efficacy and longevity of denervation procedures.

### BACKGROUND

Existing technology used for denervation primarily includes radiofrequency ablation, which is commonly performed in a monopolar configuration where current is passed between a probe and a ground pad. For example, US 2012/259269 A1 discloses a system for denervation of the renal sympathetic nerve and iontophoresis drug delivery including an iontophoresis catheter fitted with a drug coated balloon. The balloon contacts the vessel wall when inflated. One or more electrodes are associated with a surface of the balloon and may be disposed on an outer surface of the balloon. The electrodes are operably coupled to an energy source configured to produce a bipolar or monopolar electric field between two balloon electrodes and/or between one balloon electrode and another electrode placed in contact with the patient's body.

Unfortunately, nerve fibers may regenerate over time, leading to the need for repeated denervation procedures. The present disclosure pertains to devices and methods for use in inhibiting nerve regeneration after the performance of denervation procedures, including radiofrequency ablation denervation procedures.

### SUMMARY

The present invention relates to a system for denervation according to claim 1.

Said system includes a catheter including a radially expandable member, the radially expandable member including an exterior surface, a plurality of electrodes on the exterior surface configured for delivery of energy during a denervation procedure in a vessel, and a chemical agent coating on the exterior surface, wherein the chemical agent inhibits or prevents nerve regeneration. The radially expandable member is configured to have a first unexpanded configuration and a second expanded configuration and is configured to bring the exterior surface in contact with a wall of a vessel when it is in the expanded configuration. The radially expandable member is configured to contact a wall of a hepatic vessel when the radially expandable member is in the expanded configuration.

In one aspect, the system is configured for electrical current flow between the plurality of electrodes. In one aspect, the radially expandable member is a selected from a group consisting of a balloon, an elongate balloon, a shape-memory spiral, a stent, a spline structure, a basket structure, and a wireframe structure.

In one aspect, the radially expandable member is an elongate balloon and the system is configured to generate an elongate denervation treatment pattern. In one aspect, the catheter comprises an inflation lumen in fluid communication with the radially expandable member. In one aspect, the radially expandable member defines openings, the system includes a fluid for filling the inflation lumen, inflating the radially expandable member, and for dispensing through the openings in the radially expandable member.

In one aspect, the radially expandable member is a shape-memory spiral, having a first, elongate, linear configuration and a second, radially-expanded, spiral configuration.

In one aspect, the chemical agent is selected from the group consisting of inhibitors of extracellular proteins, inhibitors of neurotropic factors, inhibitors of neuropoetins, inhibitors of neurotropic factor receptors, inhibitors of cell adhesion molecules, inhibitors of cell signaling molecules, inhibitors of cell, inhibitors of cytokines and chemokines, inhibitors of sulfate proteoglycans, inhibitors of enzymes, inhibitors of arginase, inhibitors of 13-secretase, inhibitors of urokinase-type and tissue-type plasminogen activators, inhibitors of myelin-derived molecules, semaphorin-3A, paclitaxel, fibrin, brain-derived neurotrophic factor (BDNF), myelin-derived factors, phosphatase and tensin homolog (PTEN), suppressor of cytokine signaling 3 (SOCS3) gene, notch/lin12 proteins, and ZnEgr proteins.

In one aspect, the system is configured to measure impedance between combinations of the electrodes. In one aspect, the system is configured to measure impedance a reference pad on the patient's skin and one or more electrodes.

In one aspect, the radially expandable member comprises a wireframe structure having a plurality of wires extending from a proximal end to a distal end, wherein each wire including one of the electrodes, wherein each electrode defines part of the exterior surface including the chemical agent coating.

In one aspect, the radially expandable member comprises a shape-memory ribbon spiral and having an exterior side and interior side, wherein the exterior side defines the exterior surface, wherein the electrodes and the chemical agent coating are present on one side of the ribbon spiral.

A system is also described herein for denervation including a catheter including an inflation lumen and an elongate balloon in fluid communication with the inflation lumen, the elongate balloon including an exterior surface, a plurality of electrodes on the exterior surface configured for delivery of energy during a denervation procedure in a vessel, and a chemical agent coating on the exterior surface, wherein the chemical agent inhibits or prevents nerve regeneration. The elongate balloon is configured to have a first unexpanded configuration and a second expanded configuration, wherein the elongate balloon is configured to bring the exterior surface in contact with a wall of a vessel when it is in the expanded configuration.

In one aspect, the radially expandable member is configured to contact a wall of a hepatic vessel when the radially expandable member is in the expanded configuration.

Moreover, a method of treatment not covered by the wording of the claims is also described herein, said method including providing a catheter including a radially expandable member and a plurality of electrodes on an exterior surface of the radially expandable member configured for delivery of energy during a denervation procedure in a vessel. The catheter further includes a chemical agent coating on the exterior surface. The method includes expanding the radially expandable member from a first unexpanded configuration to a second expanded configuration, wherein the radially expandable member is configured to bring the exterior surface in contact with a wall of a vessel when it is in the expanded configuration. The method includes conducting a denervation procedure using electrical energy at a target site in a subject using the electrodes of the radially expandable member. The method includes introducing at least one chemical agent that inhibits or prevents nerve regeneration to the target site, wherein the chemical agent is present in the chemical agent coating on the exterior surface of the radially expandable member.

In one aspect, the denervation procedure is an irreversible electroporation procedure. In one aspect, the denervation procedure is a radiofrequency ablation (RFA) procedure.

In one aspect, conducting the denervation procedure and introducing the at least one chemical agent occur simultaneously. In one aspect, the method includes measuring impedance between combinations of the electrodes.

In one aspect, the radially expandable member contacts a wall of a hepatic vessel when the radially expandable member is in the expanded configuration.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense.

### BRIEF DESCRIPTION OF THE FIGURES

FIG. 1 is a system for denervation according to some examples including a balloon catheter.
FIG. 2 is a perspective view of a distal portion of the balloon catheter of FIG. 1 for denervation according to some examples.
FIG. 3 is a schematic view of a distal portion of the balloon catheter of FIG. 1 inserted in a hepatic vessel, according to some examples.
FIG. 4A is a side view of a shape-memory spiral in an expanded state, according to some examples.
FIG. 4B is a side view of the shape-memory spiral of FIG. 4A in an unexpanded state, according to some examples.
FIG. 4C is a perspective view of a round profile for a shape-memory spiral.
FIG. 4D is a perspective view of a ribbon profile for a shape-memory spiral.
FIG. 5A is a side view of another shape-memory spiral in an expanded state, according to some examples.
FIG. 5B is a cross-sectional view of the shape-memory spiral of FIG. 5A along line A in FIG. 5A.
FIG. 5C is a side view of another shape-memory spiral in an expanded state, according to some examples.
FIG. 5D is a cross-sectional view of the shape-memory spiral of FIG. 5C.
FIG. 6 is a schematic view of a wireframe radially expandable denervation element with a surface coated with nerve growth inhibitor according to some examples.
FIG. 7 is a schematic view of an electrode assembly for denervation according to some examples.
FIG. 8 is a schematic view of an electrode assembly for denervation according to some examples.
FIG. 9 is a schematic view of an electrode assembly for delivery of a chemical agent according to some examples.

While embodiments are susceptible to various modifications and alternative forms, specifics thereof have been shown by way of example and drawings, and will be described in detail. It should be understood, however, that the scope herein is not limited to the particular aspects described. On the contrary, the intention is to cover modifications, equivalents, and alternatives falling within the spirit and scope herein.

### DETAILED DESCRIPTION

The present disclosure provides an improved hepatic denervation therapy for metabolic disorders, in which hepatic denervation is performed using a first therapy such as radiofrequency ablation, irreversible electroporation, or other modality, and then the nerves are prevented from regenerating using a second therapy such as drug delivery of nerve growth inhibitors. Specific nerve growth inhibitors are disclosed. In some examples, the system is self-contained in a single device. In alternative examples, the system can include multiple separate devices.

The disclosed technology provides a medical treatment for medical conditions including any condition requiring the regulation of blood glucose levels, most notably diabetes, but also including insulin resistance, genetic metabolic disease, hyperglycemia, obesity, hyperlipidemia, hypertension, endocrine diseases and/or inflammatory disorders. The medical device and system including a first medical device capable of reducing sympathetic activity to control hepatic glucose production. The system also provides a nerve growth inhibitor or blocking agent. The medical device can be a catheter capable of delivering a nerve growth inhibitor or blocking agent to the treated nerve tissue to reduce the rate of nerve regeneration.

The disclosed technology provides a method that includes delivering a first treatment which at least temporarily reduces nerve signals to the liver to decrease hepatic glucose production. The method further includes delivering a second treatment which impairs the nerves' ability to regenerate and/or prevents the nerves from regaining full function. The first treatment can include hepatic denervation of nerve tissue using electrical energy, radiofrequency energy, irreversible electroporation, microwave energy, ultrasound energy, focused ultrasound (e.g. high-intensity focused ultrasound (HIFU), low-intensity focused ultrasound (LIFU)), laser energy, infrared energy, light energy, thermal energy, steam or heated water, magnetic fields, reversible electroporation, cryogenic therapy, brachytherapy, ionizing therapy, drug delivery, biologic delivery, chemical ablation (e.g. ethanol), mechanical disruption, any other therapy modality to cause disruption or modulation of the target tissue, or any combination thereof. The second treatment can include delivering a nerve growth inhibitor to the treated tissue.

In some examples, electroporation therapy is delivered concurrently with or in a time period close to the nerve growth inhibitor treatment to increase penetration of the nerve growth inhibitor into the hepatic vessel wall. In some examples, the nerve growth inhibitor and optional electroporation are delivered before, during, or after denervation therapy, such as radiofrequency ablation, irreversible electroporation, and others. In some examples, the nerve growth inhibitor and optional electroporation are delivered first via a first catheter, and then a second catheter delivers denervation therapy. In some examples, the denervation therapy is delivered first via a first catheter, and then a second catheter delivers nerve growth inhibitor and optional electroporation.

In addition or alternatively, in some example, the time period within which both denervation therapy and nerve growth inhibitor treatment are delivered is within the time period of one minimally-invasive procedure, such as within five hours, within four hours, within three hours, within two hours, within one hour, within 45 minutes, within 30 minutes or within 15 minutes.

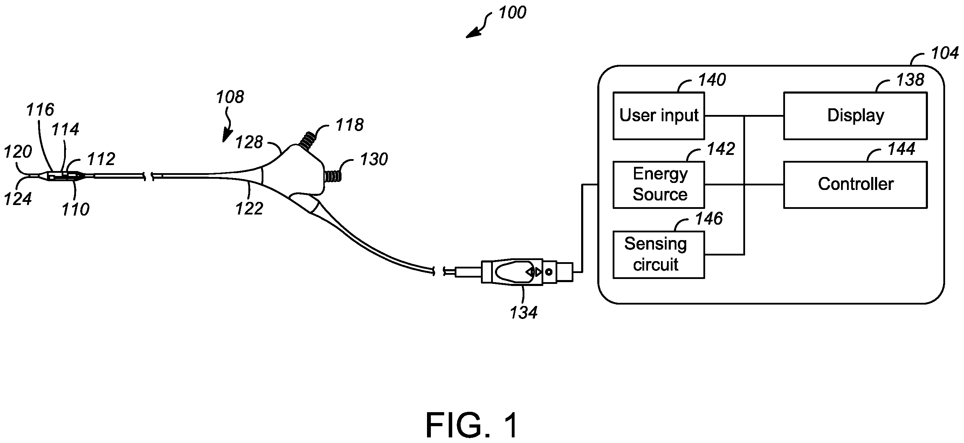

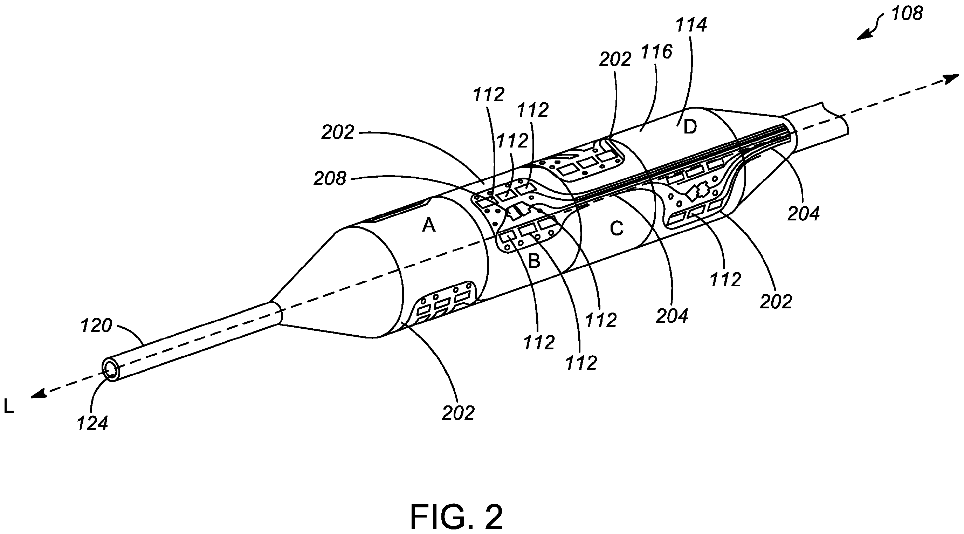

Referring now to FIG. 1, a schematic view of a denervation system is shown according to some examples. Denervation system 100 includes a generator and control device 104 and a catheter assembly 108 having a radially expandable member 110 that includes electrodes 112 on an exterior surface 114 of the radially expandable member 110. The system 100 and the electrodes 112 are configured for delivery of energy during a denervation procedure in a vessel, such as in a hepatic vessel. The system 100 can be used in other treatment areas such as another lumen or vessel of the body, including a renal vessel. The radially expandable member 110 is configured to have a first unexpanded configuration and a second expanded configuration. In the expanded configuration, the radially expandable member 110 is configured to bring the exterior surface 114 in contact with a wall of a hepatic vessel of a patient. FIG. 2 shows a closer view of the radially expandable member 110 in an expanded configuration.

In various examples, the radially expandable member 110 is an inflatable elongate balloon which moves from the unexpanded configuration to the expanded configuration by being inflated. As used herein, elongate means a structure is longer than wide. To inflate the balloon, the catheter assembly 108 includes an inflation lumen and an inflation port 118 that are in fluid communication with the balloon interior. In various examples, an inflation fluid provided to inflate the balloon can be sterile water or saline solution. In addition or alternatively, the inflation fluid may include a chemical agent that inhibits nerve growth, and that inflation fluid may be dispersed to the vessel wall, such as by weeping from openings in the balloon, to deliver the chemical agent to the lumen of the vessel.

FIG. 1 illustrates the radially expandable member as an inflatable elongate balloon. In other examples of the system of FIG. 1, the radially expandable member is a balloon, a shape-memory spiral, a stent, a basket structure, a spline structure, or a wireframe structure, and these structures may be elongate or not. These examples will be further described herein. Other types of radially expandable members can also be used with the system of FIG. 1 and with other systems described herein.

The radially expandable member 110 also includes a chemical agent coating 116 on the exterior surface, wherein the chemical agent inhibits or prevents nerve regeneration. In some examples, the chemical agent is a nerve growth inhibitor selected from the group consisting of semaphorin-3A, paclitaxel, fibrin, brain-derived neurotrophic factor (BDNF), myelin-derived factors, phosphatase and tensin homolog (PTEN), suppressor of cytokine signaling 3 (SOCS3) gene, notch/lin12 proteins, and ZnEgr proteins. Other examples of chemical agents can be used with the systems described herein, and some of these additional examples are described herein.

The catheter assembly 108 includes a catheter body 119 having a distal end 120 and a proximal end 122. The inflation lumen is a passage within the catheter body 108. The distal end 120 defines a distal guidewire port 124. The proximal end 122 of the catheter body 108 is attached to a housing 128 that includes the inflation port 118, the proximal guidewire port 130, and an electrical connector 134. The catheter body 108 may define a sheath, such that the radially expandable member 110 can be contained within the sheath when the radially expandable member is in the unexpanded state.

The electrical connector 134 connects the catheter assembly 108 to the generator and control device 104, which includes a plurality of electrical connections and is configured to deliver controlled energy to the electrodes 112. The generator and control device 104 includes a display 138, user input devices 140, an energy source 142, a controller 144, and a sensing circuit 146.

The energy source 142 may provide electrical energy, radiofrequency energy, irreversible electroporation, microwave energy, ultrasound energy, focused ultrasound (e.g. high-intensity focused ultrasound (HIFU), low-intensity focused ultrasound (LIFU)), laser energy, infrared energy, light energy, thermal energy, steam or heated water, magnetic fields, reversible electroporation, cryogenic therapy, brachytherapy, ionizing therapy, drug delivery, biologic delivery, chemical ablation (e.g. ethanol), mechanical disruption, any other therapy modality to cause disruption or modulation of the target tissue, or any combination thereof. The sensing circuit 146 can be configured to determine the impedance between combinations of electrodes. Impedance information indicates if the electrodes are in contact with a vessel wall, and indicates progress of the treatment. The display 138 may include a graphical user interface indicating which electrodes are in contact with a vessel wall, watts of energy, degrees Celsius at a temperature sensor, number of seconds of treatment time left, and which electrodes are active. User input devices 140 include buttons, switches, touch screen, a keyboard, or other devices to provide input and instructions to the generator and control device 104.

Examples of generator and control devices, energy delivery structures, electrode configurations, and energy delivery methods useable with the embodiments disclosed herein are disclosed in U.S. Pat. App. Pub. No. US 2012/0095461, entitled "Power Generating and Control Apparatus for the Treatment of Tissue", issued as U.S. Pat. No. 9,277,955 and assigned to Vessix Vascular, Inc.. Further examples are disclosed in U.S. Patent 9,037,259, entitled "Methods and apparatuses for remodeling tissue of or adjacent to a body passage", assigned to Vessix Vascular, Inc.. Further examples useable with the embodiments disclosed herein are disclosed in U.S. Pat. No. 7,742,795 entitled "Tuned RF Energy for Selective Treatment of Atheroma and Other Target Tissues and/or Structures", U.S. Pat. No. 7,291,146 entitled "Selectable Eccentric Remodeling and/or Ablation of Atherosclerotic Material", and U.S. Pub. No. 2008/0188912 entitled "System for Inducing Desirable Temperature Effects on Body Tissue", which are assigned to Vessix Vascular, Inc..

Now referring to FIG. 2, a perspective view of a distal portion of the catheter assembly 108 of FIG. 1 is shown. The radially expandable member 110, specifically an inflatable elongate balloon, includes electrodes 112 for energy delivery, according to various examples. The distal end 120 of the catheter assembly 108 defines the distal guidewire port 124. The radially expandable member 110 includes electrode pads 202 defined on its exterior surface 114, where each electrode pad 202 supports a plurality of electrodes 112, which are each electrically connected to the generator and control device 104 via conductors 204. Each electrode pad 202 also includes a temperature sensor 208.

In some examples, the electrodes 112 are arranged on the elongate balloon to form an elongate denervation treatment pattern. In some examples, the system is configured to measure impedance between combinations of the electrodes 112. As shown in FIG. 2, each electrode pad includes three electrodes 112 on a first side and three electrodes 112 on a second side. In various examples, the three electrodes 112 on one side are held at a different voltage potential than the three electrodes on the opposite side, so that an energy path is defined between the facing electrodes on the two sides of each electrode pad. The arrangements of the electrodes, the energy path formed between electrode combinations, and other aspects of the energy delivery structure options for various example are described in U.S. Patent 9,037,259 and other previously mentioned patent documents.

The exterior surface 114 is provided with a chemical agent coating 116 including a chemical agent that prevents or reduces nerve regeneration. The use of such a chemical agent can reduce the need to repeat a denervation procedure and extend the timeframe of the beneficial effects of the denervation procedure. In various examples, the chemical agent coating is present where the radially expandable member 110 is expected to contact a vessel wall when it is in an expanded configuration, such as the portion of the radially expandable member that is cylindrical and has the maximum diameter in the expanded configuration. In various examples, the chemical agent coating is present on portions of the exterior surface of the radially expandable member 110. In various examples, the chemical agent coating is not present on the electrodes 112. In various examples, the chemical agent coating is present on the portions of the radially expandable member 110 that are not occupied by the electrodes 112.

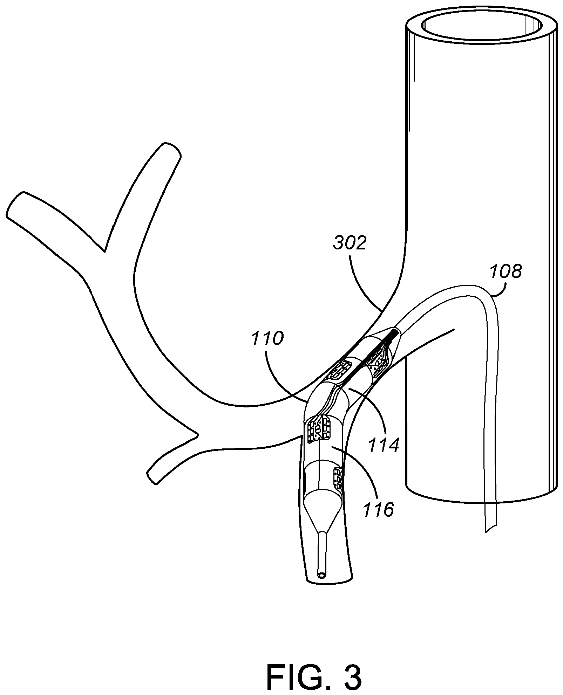

FIG. 3 is a schematic view of a catheter assembly for denervation inserted in a vessel, according to some examples. The catheter assembly 108 is shown with the radially expandable member 110 within a hepatic vessel 300, including a chemical agent coating 116 on the exterior surface 114 of the radially expandable member 110. The radially expandable member 110 is shown in an expanded configuration, so that the exterior surface 114 is brought into contact with the vessel wall. The expanded configuration brings the electrodes 112 into contact with the vessel wall to aid in the delivery of energy to damage sympathetic nerves along the vessel. The expanded configuration also brings the exterior surface 114 and the chemical agent coating 116 into contact with the vessel wall to aid transfer of the chemical agent into the vessel wall.

In some examples, the radially expandable member is a shape-memory member, having a first, elongate, linear configuration and a second, radially-expanded configuration. The shape-memory member can include or be made from shape retention material or a shape-memory material such as a shape-memory alloy. Examples of shape-memory material are nickel titanium alloy, also known as nitinol, copper aluminum nickel alloy, and stainless steel. A shape-memory material as used herein is a material that "remembers" its original shape and that when deformed returns to its pre-deformed shape. A shape retention material is a material that retains its shape once positioned.

In various examples, the radially expandable member is a shape-memory spiral, having a first, elongate, unexpanded configuration and a second, radially-expanded, spiral configuration.

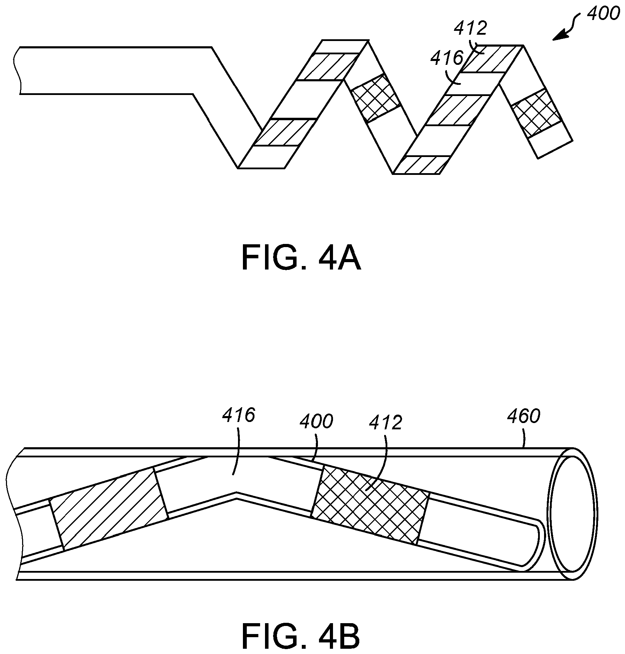

FIG. 4A is a side view of a distal end of a catheter including an embedded shape-memory to define a shape-memory spiral 400, where the shape-memory spiral is in an expanded state in FIG. 4A, according to some examples. FIG. 4B is a side view of the shape-memory spiral 400 of FIG. 4A in an unexpanded state, according to some examples. The shape-memory spiral 400 includes two or more electrodes 412. The shape-memory spiral 400 includes an exterior side and an interior side, where the exterior side defines an exterior surface. The electrodes 412 are present on the exterior surface. The exterior surface includes a chemical agent coating 416 on the exterior surface. In various examples, the chemical agent coating 416 is not present on the electrodes 412. In various examples, the chemical agent coating 416 is present on the portions of the shape-memory spiral 400 in between the electrodes 412.

In FIG. 4B, the shape-memory spiral 400 is shown in its unexpanded state constrained within a sheath 460. In one example, the shape-memory spiral 400 is delivered to the treatment site by being pushed within the sheath 460 until it arrives at a distal end of the sheath 460. The shape-memory spiral 400 is moved into its expanded configuration by pushing the shape-memory spiral 400 out of the distal end of the sheath 460, so that it protrudes from the distal end of the sheath. Once it is not constrained by the sheath 460, the shape-memory spiral 400 springs into its expanded shape as shown in FIG. 4A, in some examples.

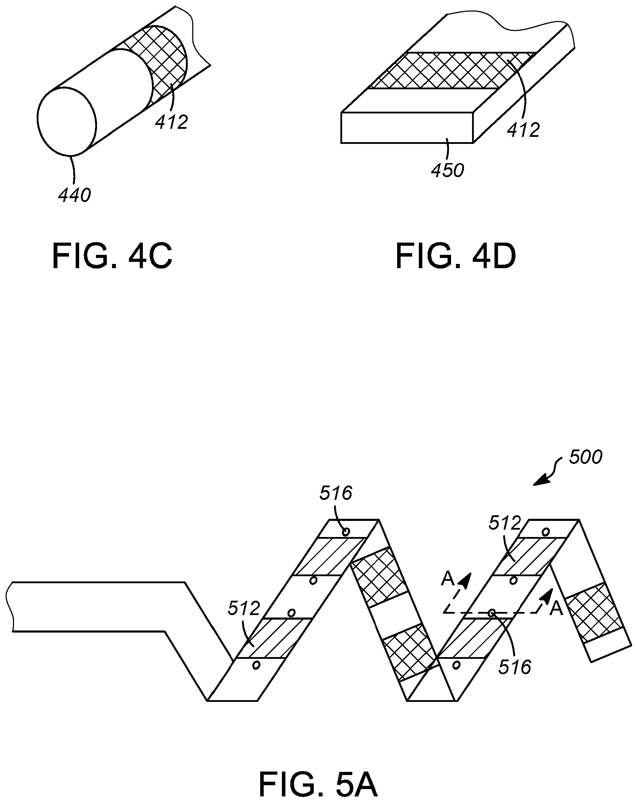

In various examples, the radially expandable shape-memory spiral member has a circular cross-section or an oblong cross-section, such as a rectangular or ribbon cross-section. FIG. 4C is a perspective view of a round profile for a shape-memory spiral 440. FIG. 4D is a perspective view of a ribbon profile for a shape-memory spiral 450.

In various examples, the diameter of the shape-memory spiral in its expanded state is sized to ensure intimal contact with the inner diameter of the lumen. Examples of diameters and other dimensions for radially expandable members such as a shape-memory spiral are provided herein.

Where the shape-memory spiral has an oblong shape such as a ribbon shape, the ribbon width, or catheter cross-sectional diameter in various examples is at least about 0.05 mm, at least about 0.3 mm, at least about 0.5 mm, at most about 5 mm, at most about 3 mm, at most about 2 mm, at least about 0.05 mm and at most about 5 i-nm, at least about 0.3 mm and at most about 3 mm, or at least about 0.5 mm and at most about 2 mm. A shape-memory spiral with a round cross-sectional shape can also have these cross-sectional diameters in various embodiments.

In various examples, the chemical agent coating is not present on the shape memory spiral. A chemical agent can be applied to the treatment area using a separate device, such as a balloon device with a coated surface or a balloon device filled with a chemical agent, to name a few examples, before or after denervation therapy.

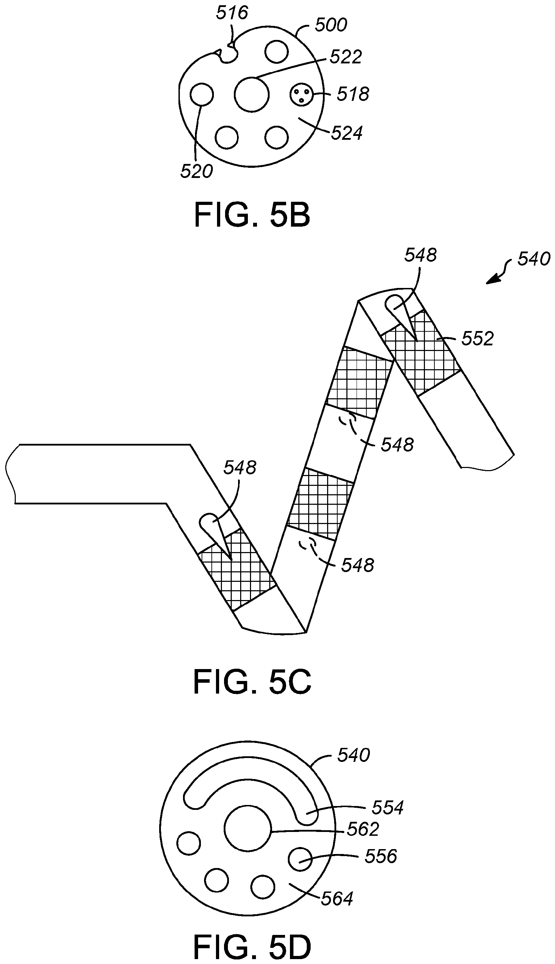

FIG. 5A illustrates another example of a distal end of a catheter having a shape-memory spiral 500, including electrodes 512 and pores 516 for delivery of a chemical agent. In various examples, the catheter has two or more lumens for delivery of a chemical agent that terminate at one of the pores 516 in the shape-memory spiral 500. FIG. 5B is a cross-sectional view of the shape-memory spiral 500 along line A in FIG. 5A. The shape-memory spiral 500 includes a pore 516 and multiple lumens 518. The lumens may define passageways for a chemical agent or may contain one or more conductors, such as shown in lumen 520, in various examples. A conductor or lead is electrically connected to each electrode. A shape-memory material, such as nitinol, may be present in a center lumen 522. The shape-memory spiral 500 may further include an insulating material 524 surrounding the shape-memory material in the center lumen 522 and defining the lumens 518 and 520.

FIG. 5C illustrates another example of a distal end of a catheter having a shape-memory spiral 540, which includes electrodes 552 and retractable needles 548 for delivery of a chemical agent. The needles 548 can advance from and retract into the exterior surface of the shape-memory spiral 540 to inject a chemical agent into tissue of a vessel. The needles 548 may pierce the adjacent tissue to a depth of about 2 mm, about 3 mm, or about 4 mm, in various embodiments. The injection of a chemical agent can occur before, during, or after denervation therapy, or combinations of these.

FIG. 5D is a cross-sectional view of the shape-memory spiral 540 and shows a lumen 554 for a retractable needle 548. The needles 548 and corresponding lumens 554 are positioned along an exterior side of the shape-memory spiral 540 in various embodiments, so that the needles 548 deploy into the tissue surface of a vessel. The shape-memory spiral 540 further includes lumens 556 for conductors, and a conductor or lead is electrically connected to each electrode. A shape-memory material, such as nitinol, may be present in a center lumen 562. The shape-memory spiral 540 may further include an insulating material 564 surrounding the center lumen 562 and defining the lumens 554 and 556.

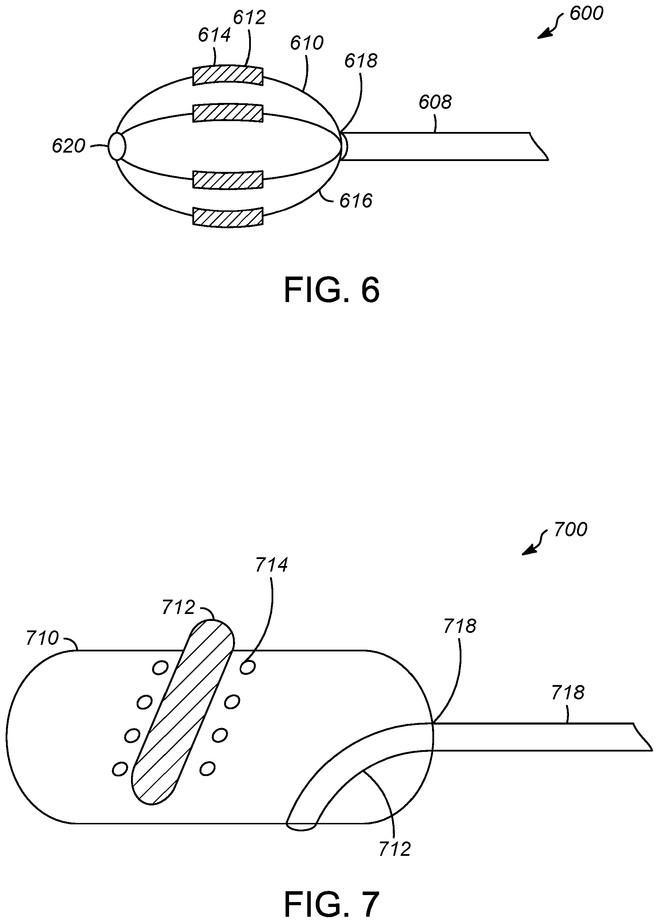

FIG. 6 is a schematic view of a denervation catheter assembly 600 with a wireframe radially expandable member 610 having electrodes 612 and an exterior surface 614 coated with a chemical agent coating including a nerve growth inhibitor according to some examples.

The wireframe structure 610 includes a plurality of wires 616 extending from a proximal end 618 of the structure to a distal end 620 of the structure. Each wire supports at least one of the electrodes. In various examples, each wire 616 supports two electrodes, three electrodes, four electrodes, five electrodes, six electrodes, or another number of electrodes. Each electrode 612 defines part of an exterior surface of the radially expandable member. Portions of the exterior surface of the radially expandable member include the chemical agent coating.

A catheter body 608 may define a sheath, such that the radially expandable member 610 can be contained within the catheter body 608 when the radially expandable member is in the unexpanded state. The wires 616 may include a shape-memory material such as nitinol. The wireframe structure 610 is formed to have an expandable basket shape in various examples. In various examples, the wireframe structure 610 is attached to a rod at its proximal end 618 within catheter body 608. In various examples, the rod can be used to push the wireframe structure 610 out of catheter body 608, causing the wireframe structure 610 to spring into its expanded shape. When the rod is pulled in a proximal direction, the wireframe structure 610 is pulled in catheter body 608, is constrained, and takes on its unexpanded shape.

In addition or alternatively, a distal rod can extend to the distal end 620 of the wireframe structure 610. The push and pull of the distal rod can cause the basket to expand and collapse. For such a design, the wires 616 may not need to rely on a shape-memory material such as nitinol, and can instead be made of a shape retention material such as stainless steel. In addition or alternatively, the wires 616 can be made of a flexible circuit. One example of such a flexible circuit is included in the INTELLAMAP ORION^{™} mapping catheter available from Boston Scientific Corporation Inc., headquartered in Marlborough, MA, USA.

In various examples, the diameter of the wireframe structure in its expanded state is sized to ensure intimal contact with the inner diameter of the lumen. Examples of diameters and other dimensions for radially expandable members such as a wireframe structure are provided herein.

FIG. 7 is a schematic view of a denervation catheter assembly 700 with an inflatable balloon 710 having an electrode strip 712 and defining pores 714 for dispensing a chemical agent including a nerve growth inhibitor according to some examples. The inflatable balloon 710 is connected to a catheter body 708 at a proximal end 718 of the inflatable balloon. The catheter body 708 may include an inflation lumen to inflate the balloon 710 from the unexpanded to the expanded configuration. The catheter body 708 may also include a sheath for the balloon 710 to be withdrawn into when the balloon 710 is in the unexpanded state.

The electrode strip 712 may include one electrode, such as for a monopolar device, or multiple electrodes. The electrode or electrodes may be configured to deliver denervation therapy to a full 360 degree circumference of the lumen or to ranges of the circumference less than that, such as to at least about 30 degrees, at least about 45 degrees, at least about 60 degrees, at least about 90 degrees, at least about 120 degrees, at least about 150 degrees, at least about 180 degrees, at most about 180 degrees, at most about 210 degrees, at most about 240 degrees, at most about 270 degrees, at least about 30 degrees and at most about 270 degrees, or at least about 45 degrees and at most about 180 degrees. A spiral configuration of the electrode strip is does not delivery therapy to the full 360 degree circumference in the same axial location, thus reducing the potential for an acute reaction causing full constriction of the vessel.

The pores 714 may a diameter ranging from a 0.1 microns to 5 millimeters. In one example, there are several hundred pores 714 defined in the inflatable balloon 710 with each pore having a diameter of about 0.5 micron. The pores may be oriented on one side of the electrode strip, on both sides of the electrode strip as shown in FIG. 7, or may be in other patterns. The pores may cover the entire outer diameter of the balloon that contacts the luminal surface of the vessel.

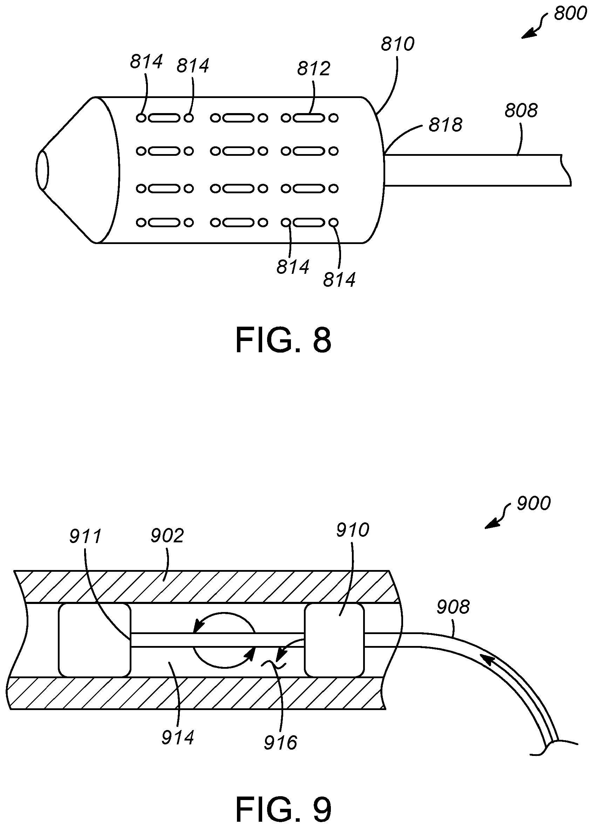

FIG. 8 is a schematic view of a denervation catheter assembly 800 with an inflatable balloon 810 having an array of electrodes 812 and micro-needles 814 for dispensing a chemical agent including a nerve growth inhibitor according to some examples. In various embodiments, holes can be provided in place of micro-needles 814, where the holes can dispense nerve growth inhibitor or a fluid.

A pair of micro-needles 814 flanks each electrode 812 in the example of FIG. 8. The micro-needles 814 can penetrate into a vessel wall to deliver the chemical agent. By increasing the depth of the delivery of the chemical agent, the effectiveness of the chemical agent against nerve regrowth may be increased. The micro-needles 814 can also be connected to a sensing circuit to detect impedance of the tissue between each pair of micro-needles 814.

The inflatable balloon 810 is connected to a catheter body 808 at a proximal end 818 of the inflatable balloon. The catheter body 808 may include an inflation lumen to inflate the balloon 810 from the unexpanded to the expanded configuration. The catheter body 808 may also include a sheath for the balloon 810 to be withdrawn into when it is in the unexpanded state.

In one example, the microneedles deploy or more fully deploy when the balloon is pressurized. In other words, the needles protrude more fully from an exterior surface of the balloon when pressure is applied internally. To avoid tissue damage during deliver of the balloon catheter, for example, snagging tissue with the needles, the balloon would be in a collapsed state and inside a delivery sheath during delivery of the balloon to the treatment site. The needles may protrude from the balloon surface in the range of 0.1 mm to 1 mm. The needles may serve to measure electrical impedance in local regions, deliver denervation therapy, such as radiofrequency energy, or both.

The balloon may include one or more needles near each electrode. In various examples, the needles are spaced apart by at least about 1 mm and at most about 10 mm. In various examples, the balloon includes at least about 2 microneedles and at most about 2000 microneedles, or at least about preferably 20 microneedles and at most about 200 microneedles, around the circumference of the balloon.

FIG. 9 is a schematic view of a catheter assembly 900 for delivery of a chemical agent deployed within a vessel 902, having a catheter body 908, first inflatable balloon 910 and a second inflatable balloon 911. The catheter assembly 900 can be used to treat a section of a vessel 902 with nerve growth inhibitor after a denervation treatment, to reduce nerve regrowth after the denervation treatment.

The catheter body 908 may include an inflation lumen to inflate the balloons 910, 911 from an unexpanded configuration to an expanded configuration. The catheter body 908 may also include a sheath for the balloons 910, 911 to be withdrawn into when they are in the unexpanded state. After the first and second balloons are deployed, spaced away from each other, and inflated, a nerve growth inhibitor 916 can be injected into and circulated within the space 914 in between the balloons. Residual nerve growth inhibitor can be removed before deflating the balloons.

In various examples, the diameter of the balloons when expanded is at least about 2 mm, at least about 3 mm, at most about 10 mm, at most about 5 mm, at least about 2 mm and at most about 10 mm, or at least about 3 mm and at most about 5 mm. A distance between the balloons can range from at least about 10 mm to at least about 50 mm.

The catheter assembly 900 can be configured to delivery electrical energy, for example, electroporation, to tire fluid between the two balloons, to increase permeability of the nerve growth inhibitor into the vessel wall. The balloon material may include polyethylene teraphthalate (PET), a thermoplastic elastomer, such as PEBAX^{™} thermoplastic elastomer available from Arkema, having a business location in King of Prussia, Pennsylvania, USA, nylon, non-compliant balloon materials, and semi-compliant balloon materials.

### Methods of Denervation Treatment

A method of treatment according to various examples described herein includes providing a catheter including a radially expandable member and a plurality of electrodes on an exterior surface of the radially expandable member. The electrodes are configured for delivery of energy during a denervation procedure in a vessel. The radially expandable member also includes a chemical agent coating on the exterior surface.

The system may include a guidewire, and the catheter may include a guidewire passage. The catheter can be introduced to a treatment site in a patient's body by advancing the guidewire to the treatment site, advancing the catheter assembly over the guidewire until the distal end of the catheter assembly is near the treatment site, and advancing the unexpanded radially expandable member out of the catheter sheath. Other techniques for positioning the radially expandable member at the treatment site can also be used.

The radially expandable member is expanded from a first unexpanded configuration to a second expanded configuration. Many different types of radially expandable members can be used in this step, such as the examples described herein and in the mentioned documents. The radially expandable member is configured to bring the exterior surface in contact with a wall of a vessel when it is in the expanded configuration. In one example, the radially expandable member is configured to bring the exterior surface in contact with a wall of a hepatic vessel of a patient when it is in the expanded configuration.

A denervation procedure is conducted using electrical energy at a target site in a subject using the electrodes of the radially expandable member. The denervation procedure can be a radiofrequency ablation procedure, an irreversible electroporation procedure, or another denervation procedure including the other examples described herein and in the mentioned documents.

In various examples, the treatment site is within a vessel, lumen or other vasculature of a patient. In various examples, the radially expandable member is advanced to the treatment site through the vasculature of the patient. In other words, the radially expandable member is intravascularly delivered.

At least one chemical agent that inhibits or prevents nerve regeneration is introduced to the target site close in time to the denervation procedure. In various examples, the chemical agent is present in a chemical agent coating on the exterior surface of the radially expandable member. In other examples, there is no chemical agent coating on the exterior surface, and the chemical agent is injected into the vessel wall or circulated in the vessel at the target site. In some examples, a single catheter assembly is configured to perform the denervation procedure and deliver the chemical agent. In other examples, one catheter assembly provides the denervation procedure and a different catheter assembly delivers the chemical agent.

In some examples, the chemical agent is delivered after the denervation therapy. In some examples, the chemical agent is delivered simultaneous with the denervation therapy. In some examples, the nerve growth inhibitor contacts the surface of the vessel wall before denervation therapy is delivered. For example, in the case of a drug-coated balloon having electrodes on its surface, the drug would contact the inner surface of the vessel as soon as the balloon is inflated, before the denervation therapy is applied

The method may also include measuring impedance between combinations of individual electrodes. The method may also include measuring impedance between one or more electrodes and a reference ground pad located on a patient's skin. For example, the system may measure impedance between a first electrode and a ground pad, between a second electrode and a ground pad, or both. Measurement of impedance can provide information about the progress of the denervation procedure, the contact of electrodes with the vessel wall, and other information.

### Radially Expandable Member Dimensions and Materials

In various examples, the diameter of a radially expandable member in its expanded state is at least about 1 mm, at least about 2 mm, at least about 3 mm, at most about 20 mm, at most about 10 mm, at most about 7 mm, at least about 1 mm and at most about 20 mm, at least about 2 mm and at most about 10 mm, or at least about 3 mm and at most about 7 mm.

In various examples, the length of a radially expandable member in its expanded state is at least about 10 mm, at least about 20 mm, at most about 30 mm, at most about 50 mm, at least about 10 mm and at most about 50 mm, at least about 20 mm and at most about 30 mm, or about 25 mm.

Where the radially expandable member is a balloon, the balloon material may include polyethylene teraphthalate (PET), a thermoplastic elastomer, such as PEBAX^{™} thermoplastic elastomer available from Arkema, having a business location in King of Prussia, Pennsylvania, USA, nylon, non-compliant balloon materials, and semi-compliant balloon materials.

### Examples of Nerve Growth Inhibitors

The nerve growth inhibitor used with any of the example medical devices described herein can include one of the following or a combination of the following agents. In various examples, the nerve growth inhibitor can be provided as a coating, as a liquid, or can be encapsulated in microparticles or nanoparticles within a biodegradable shell. Encapsulated configurations can allow modulated release of the nerve growth inhibitor over the course of up to three months.

### Classes of Nerve Growth Inhibitors

Classes of nerve growth inhibitors include inhibitors of extracellular proteins such as laminin, fibronectin, tenascin, fibrinogen, and fibrin. The nerve growth inhibitors can include inhibitors of neurotropic factors such as nerve growth factor (NGF), brain-derived neurotrophic factor (BDNF), neurotrohin-3 (NT-3), NT4-5, or glial-derived nerve growth factor (GDNF). The nerve growth inhibitors can include inhibitors of neuropoetins such as leukemia inhibitory factor (LIF) and ciliary neurotrophic factor (CNTF), oncostatin M (OSM), and interleukin 6 (IL-6).

The nerve growth inhibitors can be inhibitors of neurotropic factor receptors, such as tyrosine kinase receptors (TrkA, TrkB and TRk C), common neurotrophic receptor (P75NTR), ErbB receptors, or fibroblast growth factor receptors. The nerve growth inhibitors can be inhibitors of cell adhesion molecules (CAM), such as N-CAM, Ng-CAM/L1, N-cadherin and L2-HWk-1. The nerve growth inhibitors can be inhibitors of cell signaling molecules such as Ras, Phosphotidyl Inositol 3-kinase, Phospholipase c- gamma 1, Mitogen Activated Phospho kinase, Protein Kinase A, Janus kinases (JAKs), Signal Transducer and Activator of Transcription proteins (Jaks/STATs signaling molecules). Kinase inhibitors include staurosporine, II 89, dihydrochloride, Cyclic Adenosine Monophophate-Rp (cAMPS.-Rp), triethylammonium salt, KT 5720, wortmannin, LY294002, 1C486068, 187114, GDC-0941, Gefitinib, Erlotinib, Lapatinib, AZ623, K252a, KT-5555, Cyclotraxin-B, Lestaurtinib, Tofacitinib, Ruxolitinib, SB1518, CYT387, LY3009104, TG101348, WP-1034, PD173074, and Sprouty RTK Signaling Antagonist 4) (SPRY4). The nerve growth inhibitors can be inhibitors of cytokines and chemokines, which include interleukin-6, leukemia inhibitor factor, transforming growth factor 131, and monocyte-chemotactic protein 1.

The nerve growth inhibitors can be inhibitors of sulfate proteoglycans, such as keratin sulfate proteoglycans. The nerve growth inhibitors can be inhibitors of chondroitin sulfate proteoglycans, such as neurocan, brevican, versican, phosphacan, aggrecan, and NG2. The nerve growth inhibitors can be inhibitors of enzymes, including the enzymes Arginase 1, Chondroitinase ABC, 13-secretase BACE1, urokinase-type plasminogen activator, and tissue-type plasminogen activator. The nerve growth inhibitors can be inhibitors of arginase, including N-hydroxy-L-arginine and 2(S)-amino-6-boronohexonic acid. The nerve growth inhibitors can be inhibitors of 13-secretase, such as N-Benzyloxycarbonyl-Val-Leu-leucinal, 11-Glu -Val-Asn-Statine-Val-Ala-Glu-Phe-NH2, and H-Lys-Thr-Glu-Glu-Ile-Ser-Glu-Val-Asn-Stat-Val-Ala-Glu-Phe-OH. The nerve growth inhibitors can be inhibitors of urokinase-type and tissue-type plasminogen activators, including serpin El, Tiplaxtinin, and plasminogen activator inhibitor-2.

The nerve growth inhibitors can be inhibitors of myelin-derived molecules, such as myelin-associated glycoprotein, oligodendrocyte myelin glycoprotein, Nogo-A/B/C, Semaphorin 4D, Semaphorin 3A, and ephrin-B3.

### Specific Examples of Nerve Growth Inhibitors

The nerve growth inhibitor used in conjunction with the first medical treatment can include Paclitaxel, Semaphorin-3A, Fibrin, Brain-derived neurotrophic factor (BDNF), Myelin-derived factors including NogoR and PirB, PTEN (dual phosphate and tensin homolog), SOCS3, Notch signaling (Notch/lin12), and ZnEgr.

### Carriers and Excipients combined with nerve growth inhibitors

Carriers, excipients, or both can be combined with nerve growth inhibitors used with the systems describe herein. Carriers could produce a delayed release, for example such that the nerve growth inhibitor is released over a time course such as days, weeks, or months. In various examples, a modulated release of a nerve growth inhibitor occurs over about one week, two weeks, three weeks, four weeks, one month, two months, three months, four months, five months, or six months.

In various examples, a nerve growth inhibitor is encapsulated in microparticles, such as microspheres or nanoparticles, within a biodegradable shell or matrix allowing a modulated release of the nerve growth inhibitor. In various examples, microparticles can be solid with the drug intermixed with the microparticle material. Biostable or biodegradable polymer are examples of a microparticle material. The microparticles can be injected into a vessel wall or target tissue. Microparticle material can be a biodegradable matrix material such as poly-lactic-co-glycolic acid (PLGA), polylactic acid (PLA), poly-L-lactic acid (PLLA), or other biostable or biodegradable polymers.

Some excipients could help in transporting a nerve growth inhibitor deeper into a vessel wall, such as a hepatic vessel wall. The advantage obtained is similar that achieved with electroporation. In various examples, the nerve growth inhibitor is combined with an excipient that facilitates transfer across the luminal surface of the hepatic artery or another vessel and to the target nerve location. Exemplary excipients include polysorbate, sorbitol, urea, iopromide, citrate ester excipient, as used for example in TransPax^{™} coating available from Boston Scientific Corporation Inc., headquartered in Marlborough, MA, USA, butyryl-tri-hexyl citrate (BTHC), shellac, or keratose hydrogel.

### Denervation Procedure Examples

The first treatment can include hepatic denervation of nerve tissue using electrical energy, radiofrequency energy, irreversible electroporation, microwave energy, ultrasound energy, focused ultrasound (e.g. high-intensity focused ultrasound (HIFU), low-intensity focused ultrasound (LIFU), laser energy, infrared energy, light energy, thermal energy, steam or heated water, magnetic fields, reversible electroporation, cryogenic therapy, brachytherapy, ionizing therapy, drug delivery, biologic delivery, chemical ablation (e.g. ethanol), mechanical disruption, any other therapy modality to cause disruption or modulation of the target tissue, or any combination thereof.

During delivery of denervation therapy, such as radiofrequency ablation, saline or another fluid can be delivered to cool the inner surface of the lumen. Such cooling fluid can be provided to the inner lumen surface simultaneously with delivering nerve growth inhibitor.

### Parameters for Radiofrequency Ablation

In various examples, such as using the radially expandable member of FIG. 2, the energy has a frequency about 20 kHz to 5 MHz, or about 400 kHz to 500 kHz, for example 460 kHz. The target temperature for the embedded temperature sensor is about 60 °C to 95 °C, or about 80 °C. The duration of radiofrequency energy application ranges from about 10 seconds to 5 minutes, or ranges from about 30 seconds to 2 minutes.

The energy can be applied (1) simultaneously, (2) sequentially or (3) in a time switching manner. A time switching manner means the energy is applied to one or more selected electrodes for a short period, such as 20 milliseconds, and then energy is applied to one or more other selected electrodes for another short period. The energy application switches quickly among electrodes.

### Parameters for Irreversible Electroporation

For irreversible electroporation, the pulse width ranges from 10 nanoseconds to 1 millisecond, or 1 microsecond to 75 microseconds. The pulse can be either biphasic, having both positive and negative phases, or monophasic. The voltage ranges from 200 V to 5000 V, or 1000 V to 3000 V, to have the electrical field strength in tissue from 500 V/cm to 2000 V/cm, such as 1000 V/cm to 1500 V/cm to cause cell damage.

### Parameters for Electroporation to Facilitate Drug Penetration

For reversible electroporation, the pulse width ranges from 10 nanoseconds to 1 milliseconds, preferably 1 microsecond to 75 microsecond. The pulse can be either biphasic, having both positive and negative phases, or monophasic. The voltage ranges from 50 V to 5000 V, such as 100 V to 3000 V, to have the electrical field strength in tissue from 50 V/cm to 800 V/cm, such as 100 V/cm to 400 V/cm, to cause reversible cell damage to allow drug to penetrate the membrane.

It should be noted that, as used in this specification and the appended claims, the singular forms "a," "an," and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as arranged and configured, constructed and arranged, constructed, manufactured and arranged, and the like.

## Claims

1. A system for denervation (100) comprising a catheter (108) comprising a radially expandable member (110; 610; 710), the radially expandable member (110; 610; 710) comprising:
an exterior surface (114);
a plurality of electrodes (112; 412; 512; 812) on the exterior surface (114) configured for delivery of energy during a denervation procedure in a vessel; and
a chemical agent coating (116; 416) on the exterior surface (114), wherein the chemical agent inhibits or prevents nerve regeneration;
wherein the radially expandable member (110; 610; 710) is configured to have a first unexpanded configuration and a second expanded configuration, wherein the radially expandable member (110; 610; 710) is configured to bring the exterior surface (114) in contact with a wall of a vessel when it is in the expanded configuration, and wherein the radially expandable member (110; 610; 710) is configured to contact a wall of a hepatic vessel when the radially expandable member (110; 610; 710) is in the expanded configuration.

2. The system of claim 1 where the system is configured for electrical current flow between the plurality of electrodes (112; 412; 512; 812).

3. The system of any of claims 1-2 wherein the radially expandable member (110; 610; 710) is a selected from a group consisting of a balloon, an elongate balloon, a shape-memory spiral, a stent, a spline structure, a basket structure, and a wireframe structure.

4. The system of any of claims 1-2 wherein the radially expandable member (110; 610; 710) is an elongate balloon and the system is configured to generate an elongate denervation treatment pattern.

5. The system of any of claims 1-4 wherein the catheter comprises an inflation lumen in fluid communication with the radially expandable member (110; 610; 710).

6. The system of claim 5 wherein the radially expandable member (110; 610; 710) defines openings, further comprising a fluid for filling the inflation lumen, inflating the radially expandable member (110; 610; 710), and for dispensing through the openings in the radially expandable member (110; 610; 710).

7. The system of any of claims 1-2 wherein the radially expandable member (110; 610; 710) is a shape-memory spiral, having a first, elongate, linear configuration and a second, radially-expanded, spiral configuration.

8. The system of any of claims 1-7 wherein the chemical agent is selected from the group consisting of inhibitors of extracellular proteins, inhibitors of neurotropic factors, inhibitors of neuropoetins, inhibitors of neurotropic factor receptors, inhibitors of cell adhesion molecules, inhibitors of cell signaling molecules, inhibitors of cell, inhibitors of cytokines and chemokines, inhibitors of sulfate proteoglycans, inhibitors of enzymes, inhibitors of arginase, inhibitors of 13-secretase, inhibitors of urokinase-type and tissue-type plasminogen activators, inhibitors of myelin-derived molecules, semaphorin-3A, paclitaxel, fibrin, brain-derived neurotrophic factor (BDNF), myelin-derived factors, phosphatase and tensin homolog (PTEN), suppressor of cytokine signaling 3 (SOCS3) gene, notch/lin12 proteins, and ZnEgr proteins.

9. The system of any of claims 1-8 wherein the system is configured to measure impedance between combinations of the electrodes (112; 412; 512; 812).

10. The system of any of claims 1-9 wherein the radially expandable member comprises a wireframe structure having a plurality of wires extending from a proximal end to a distal end, each wire comprising one of the electrodes, wherein each electrode defines part of the exterior surface including the chemical agent coating.

11. The system of any of claims 1-10 wherein the radially expandable member comprises a shape-memory ribbon spiral and having an exterior side and interior side, wherein the exterior side defines the exterior surface, wherein the electrodes and the chemical agent coating are present on one side of the ribbon spiral.

## Patentansprüche

1. System zur Denervierung (100), mit einem Katheter (108), der ein radial ausdehnbares Element (110; 610; 710) aufweist, wobei das radial ausdehnbare Element (110; 610; 710) aufweist:
eine Außenfläche (114),
mehrere Elektroden (112; 412; 512; 812) auf der Außenfläche (114), die dafür konfiguriert sind, während eines Denervierungsvorgangs in einem Gefäß Energie zuzuführen; und
eine Beschichtung (116; 416) mit einer chemischen Substanz auf der Außenfläche (114), wobei die chemische Substanz die Nervenregeneration hemmt oder verhindert,
wobei das radial ausdehnbare Element (110; 610; 710) derart konfiguriert ist, dass es eine erste, nicht ausgedehnte Konfiguration und eine zweite, ausgedehnte Konfiguration aufweist, wobei das radial ausdehnbare Element (110; 610; 710) dafür konfiguriert ist, die Außenfläche (114) in Kontakt mit einer Wand eines Gefäßes zu bringen, wenn es sich in der ausgedehnten Konfiguration befindet, und wobei das radial ausdehnbare Element (110; 610; 710) dafür konfiguriert ist, mit einer Wand eines Lebergefäßes in Kontakt zu kommen, wenn sich das radial ausdehnbare Element (110; 610; 710) in der ausgedehnten Konfiguration befindet.

2. System nach Anspruch 1, wobei das System für einen elektrischen Stromfluss zwischen den mehreren Elektroden (112; 412; 512; 812) konfiguriert ist.

3. System nach Anspruch 1 oder 2, wobei das radial ausdehnbare Element (110; 610; 710) ausgewählt ist aus einer Gruppe bestehend aus einem Ballon, einem länglichen Ballon, einer Formgedächtnisspirale, einem Stent, einer Spline-Struktur, einer Korbstruktur und einer Drahtgitterstruktur.

4. System nach Anspruch 1 oder 2, wobei das radial ausdehnbare Element (110; 610; 710) ein länglicher Ballon ist und das System dafür konfiguriert ist, ein längliches Denervierungsbehandlungsmuster zu erzeugen.

5. System nach einem der Ansprüche 1 bis 4, wobei der Katheter ein Befüllungslumen aufweist, das mit dem radial ausdehnbaren Element (110; 610; 710) in Fluidverbindung steht.

6. System nach Anspruch 5, wobei das radial ausdehnbare Element (110; 610; 710) Öffnungen definiert, ferner mit einem Fluid zum Füllen des Befüllungslumens, Erweitern des radial ausdehnbaren Elements (110; 610; 710) und zum Abgeben des Fluids über die Öffnungen in dem radial ausdehnbaren Element (110; 610; 710).

7. System nach Anspruch 1 bis 2, wobei das radial ausdehnbare Element (110; 610; 710) eine Formgedächtnisspirale ist, die eine erste, längliche, lineare Konfiguration und eine zweite, radial ausgedehnte, spiralförmige Konfiguration aufweist.

8. System nach einem der Ansprüche 1 bis 7, wobei die chemische Substanz ausgewählt ist aus der Gruppe bestehend aus Inhibitoren extrazellulärer Proteine, Inhibitoren neurotroper Faktoren, Inhibitoren von Neuropoetinen, Inhibitoren von Rezeptoren für neurotrope Faktoren, Inhibitoren von Zelladhäsionsmolekülen, Inhibitoren von Zellsignalmolekülen, Zellinhibitoren, Inhibitoren von Zytokinen und Chemokinen, Inhibitoren von Sulfatproteoglykanen, Inhibitoren von Enzymen, Inhibitoren von Arginase, Inhibitoren von 13-Sekretase, Inhibitoren von Plasminogenaktivatoren vom Urokinasetyp und Gewebetyp, Inhibitoren von Myelin gewonnenen Molekülen, Semaphorin-3A, Paclitaxel, Fibrin, hirnabgeleiteter neurotropher Faktor (BDNF), von Myelin gewonnenen Faktoren, Phosphatase und Tensin-Homolog (PTEN), Suppressor of Cytokine Signaling 3 (SOCS-3) -Gen, Notch/Lin12-Proteinen und ZnEgr-Proteinen.

9. System nach einem der Ansprüche 1 bis 8, wobei das System dafür konfiguriert ist, die Impedanz zwischen Kombinationen der Elektroden (112; 412; 512; 812) zu messen.

10. System nach einem der Ansprüche 1 bis 9, wobei das radial ausdehnbare Element eine Drahtrahmenstruktur mit mehreren Drähten aufweist, die sich von einem proximalen Ende zu einem distalen Ende erstrecken, wobei jeder Draht eine der Elektroden aufweist, wobei jede Elektrode einen Teil der Außenfläche definiert, die die Beschichtung mit der chemischen Substanz enthält.

11. System nach einem der Ansprüche 1 bis 10, wobei das radial ausdehnbare Element eine Formgedächtnisbandspirale aufweist und eine Außenseite und eine Innenseite aufweist, wobei die Außenseite die Außenfläche definiert, wobei die Elektroden und die Beschichtung mit der chemischen Substanz auf einer Seite der Bandspirale vorhanden sind.

## Revendications

1. Système de dénervation (100) comprenant un cathéter (108) comprenant un élément radialement extensible (110 ; 610 ; 710), l'élément radialement extensible (110 ; 610 ; 710) comprenant :
une surface extérieure (114) ;
une pluralité d'électrodes (112 ; 412 ; 512 ; 812) sur la surface extérieure (114) configurées pour la distribution d'énergie pendant une procédure de dénervation dans un vaisseau ; et
un revêtement d'agent chimique (116 ; 416) sur la surface extérieure (114), dans lequel l'agent chimique inhibe ou empêche la régénération nerveuse ;
dans lequel l'élément radialement extensible (110 ; 610 ; 710) est configuré pour avoir une première configuration non déployée et une deuxième configuration déployée, dans lequel l'élément radialement extensible (110 ; 610 ; 710) est configuré pour amener la surface extérieure (114) en contact avec une paroi d'un vaisseau lorsqu'il est dans la configuration déployée, et dans lequel l'élément radialement extensible (110 ; 610 ; 710) est configuré pour entrer en contact avec une paroi d'un vaisseau hépatique lorsque l'élément radialement extensible (110 ; 610 ; 710) est dans la configuration déployée.

2. Système selon la revendication 1, où le système est configuré pour que le courant électrique circule entre la pluralité d'électrodes (112 ; 412 ; 512 ; 812).

3. Système selon l'une des revendications 1 à 2, dans lequel l'élément radialement extensible (110 ; 610 ; 710) est choisi dans un groupe constitué d'un ballon, d'un ballon allongé, d'une spirale à mémoire de forme, d'un stent, d'une structure cannelée, d'une structure en panier, et d'une structure filaire.

4. Système selon l'une des revendications 1 à 2, dans lequel l'élément radialement extensible (110 ; 610 ; 710) est un ballon allongé et le système est configuré pour générer un schéma de traitement de dénervation allongé.

5. Système selon l'une des revendications 1 à 4, dans lequel le cathéter comprend une lumière de gonflage en communication fluidique avec l'élément radialement extensible (110 ; 610 ; 710).

6. Système selon la revendication 5, dans lequel l'élément radialement extensible (110 ; 610 ; 710) définit des ouvertures, comprend en outre un fluide pour remplir la lumière de gonflage, gonfler l'élément radialement extensible (110 ; 610 ; 710), et pour être distribué à travers les ouvertures dans l'élément radialement extensible (110 ; 610 ; 710).

7. Système selon l'une des revendications 1 à 2, dans lequel l'élément radialement extensible (110 ; 610 ; 710) est une spirale à mémoire de forme, ayant une première configuration linéaire allongée et une deuxième configuration en spirale déployée radialement.

8. Système selon l'une des revendications 1 à 7, dans lequel l'agent chimique est choisi dans le groupe constitué d'inhibiteurs de protéines extracellulaires, d'inhibiteurs de facteurs neurotropes, d'inhibiteurs de neuropoétines, d'inhibiteurs de récepteurs de facteurs neurotropes, d'inhibiteurs de molécules d'adhésion cellulaire, d'inhibiteurs de molécules de signalisation cellulaire, d'inhibiteurs de cellules, d'inhibiteurs de cytokines et de chimiokines, d'inhibiteurs de protéoglycanes sulfatés, d'inhibiteurs d'enzymes, d'inhibiteurs d'arginase, d'inhibiteurs de 13-sécrétase, d'inhibiteurs d'activateurs du plasminogène de type urokinase et de type tissulaire, d'inhibiteurs de molécules dérivées de la myéline, sémaphorine-3A, paclitaxel, fibrine, facteur neurotrophique dérivé du cerveau (BDNF), facteurs dérivés de la myéline, homologue de la phosphatase et de la tensine (PTEN), gène du suppresseur de la signalisation des cytokines 3 (SOCS3), protéines Notch/lin12, et protéines ZnEgr.

9. Système selon l'une des revendications 1 à 8, dans lequel le système est configuré pour mesurer l'impédance entre des combinaisons d'électrodes (112 ; 412 ; 512 ; 812).

10. Système selon l'une des revendications 1 à 9, dans lequel l'élément radialement extensible comprend une structure filaire ayant une pluralité de fils s'étendant d'une extrémité proximale à une extrémité distale, chaque fil comprenant l'une des électrodes, dans lequel chaque électrode définit une partie de la surface extérieure comportant le revêtement d'agent chimique.

11. Système selon l'une des revendications 1 à 10, dans lequel l'élément radialement extensible comprend une spirale de ruban à mémoire de forme et ayant un côté extérieur et côté intérieur, dans lequel le côté extérieur définit la surface extérieure, dans lequel les électrodes et le revêtement d'agent chimique sont présents sur un côté de la spirale de ruban.
